# EUROPEAN PATENT SPECIFICATION

(11) **EP 0 662 941 B1**
(45) Date of publication and mention of the grant of the patent: **10.12.1997**
(21) Application number: 93920064.8
(22) Date of filing: 13.08.1993
(51) Int. Cl.: C07C 17/00, C07C 1/26

(54) **PROCESSES FOR CONVERTING CHLORINATED ALKANE BYPRODUCTS OR WASTE PRODUCTS TO USEFUL, LESS CHLORINATED ALKENES**
VERFAHREN ZUR UMWANDLUNG CHLORINIERTER ALKANE, DIE ALS NEBEN- ODER ABFALLPRODUKTE ANFALLEN, IN NÜTZLICHE, NIEDRIGER CHLORIERTE ALKENE
PROCEDE DE CONVERSION DE DECHETS OU DE SOUS-PRODUITS A BASE D'ALCANE CHLORE EN ALCENES UTILES ET MOINS CHLORES

(30) Priority: 01.10.1992 US 955173
(43) Date of publication of application: 19.07.1995
(73) Proprietor: THE DOW CHEMICAL COMPANY, Midland, Michigan 48674 (US)
(72) Inventor: ITO, Larry, N., Midland, MI 48640 (US); HARLEY, A., Dale, Baton Rouge, LA 70816 (US); HOLBROOK, Michael, T., Baton Rouge, LA 70808 (US); SMITH, David, D., Baton Rouge, LA 70809 (US); MURCHISON, Craig, B., Midland, MI 48640 (US); CISNEROS, Mark, D., Baton Rouge, LA 70810 (US)
(74) Representative: Burford, Anthony Frederick
(86) International application number: US9307661
(87) International publication number: WO9407827

(56) References cited:
- EP-A- 0 015 665
- DD-A- 235 630
- FR-A- 2 409 974
- GB-A- 1 400 529
- US-A- 2 886 605

## Description

The present invention relates generally to processes for converting chlorinated alkanes, and especially chlorinated alkanes produced as byproducts or waste products from chemical manufacturing processes, to their corresponding less chlorinated alkenes which are useful in the same or different processes or saleable.

EP-A-0496446, published on July 29, 1992, describes the preparation of chlorotrifluoroethylene and trifluoroethylene from 1,1,2-trichloro-1,2,2-trifluoroethane via a catalyst comprised of copper and a Group VIII metal (palladium and platinum being preferred) on a carbon support. Several prior publications address the same conversion and describe the same or different catalysts, see, for example EP-B-0253410, EP-B-0355907 and EP-A-0459463. None of these publications describes or suggests, however, that other halogenated feedstocks might be suitably catalytically converted to less halogenated and more useful or more saleable materials, and in particular none of these references suggest that the catalysts described therein might be useful outside of the fluorocarbon art.

A seemingly separate course of development has occurred with respect to the class of chlorinated hydrocarbon feedstocks which are exclusive of the chlorofluorocarbon feedstocks (and which will henceforth and for simplicity be referred to generically as the chlorinated hydrocarbon feedstocks).

GB-A-1400529 discloses the catalytic conversion of hydrocarbon chlorides into chloride-free hydrocarbons in which the hydrocarbon chloride is reacted in the gaseous phase at 50 to 500°C in the presence of hydrogen on a rhodium-containing catalyst. The catalyst can be supported and can contain other metals or metal compounds, such as palladium, platinum, ruthenium, iridium, iron, cobalt, nickel, copper, gold, vanadium, chrominum, molybdenium and tungsten. The only exemplified catalyst containing an additional Group IB metal consist of rhodium and gold on an aluminium oxide support.

EP-A-0015665 describes the conversion of 1,1,2-trichloroethane to ethylene and vinyl chloride via a catalyst including a noble metal chloride, an alkali metal chloride, iron chloride and optionally copper chloride. Other known references relate particularly to the conversion of 1,2-dichloropropane (a byproduct of the chlorohydrin process for making propylene oxide, and hereafter referred to as PDC) to the less saturated, less chlorinated propylene (a product which can be recycled to the chlorohydrin process for making propylene oxide, used in the production of allyl chloride or put to other beneficial uses or sold).

DD-A-235,630 ("DE '630") discloses the conversion of PDC to propylene in a catalytic gas phase reaction at temperatures ranging from 170 degrees Celsius to 450 degrees Celsius. The catalyst is described as an activated carbon which has been treated with a suspension of iron oxides and/or iron oxide hydrates, and then dried at temperatures in the range of 80 degrees to 200 degrees Celsius.

Other methods described in DE '630 include the conversion (preferably at 180-250 degrees Celsius) in the presence of hydrogen and of a rhodium catalyst of PDC to propylene, the dechlorination at normal temperatures of PDC to a mixture (9:1) of propylene and chloropropylene in the presence of a pure titanium catalyst, and the reductive dehalogenation with sodium sulfide and a phase transfer catalyst of chlorinated hydrocarbons to alkylenes. The production of alkylenes from halogenated phosphonate esters under the influence of sulfur and olefinating agents containing phosphorus is also described.

Applicants' inventive processes provide for the catalytic conversion of less-desired chlorinated alkanes to reaction products including their corresponding, less-chlorinated alkenes in commercially substantial proportions (that is, at yields (defined as the selectivity to a desired product multiplied by the conversion, on an hydrogen chloride- and hydrogen-free basis) of at least 10 percent, but more preferably at least 20 percent and most preferably at least 30 percent), in which a chlorinated alkane feedstock is reacted with hydrogen in the presence of a catalyst including a selected Group IB metal or metals in an elemental or compound form with a selected Group VIII metal or metals, also in an elemental or compound form. The desired alkenes are then conventionally separated from other materials in the product stream, and may be further processed in a conventional, known manner to be placed in condition for an appropriate use or for sale.

"Less chlorinated", as used above and elsewhere herein, embraces still-chlorinated alkenes as well as alkenes having no remaining chlorine atoms associated therewith.

The present invention embraces the catalytic conversion of a wide array of possible chlorinated alkane starting materials to reaction products including the corresponding less chlorinated alkenes. Preferred chlorinated alkane feedstocks and reaction products include, for example: PDC and propylene; 2-chloropropane and propylene; 1,2,3-trichloropropane and allyl chloride, propylene; 1,1,2-trichloroethane and vinyl chloride; and, 1,1,1,2-tetrachloroethane and vinylidene chloride. A particularly preferred application is for the gaseous-phase conversion of PDC to propylene.

The catalysts employed for the various processes contemplated herein consist of, one or more Group IB metals in elemental or compound form and one or more Group VIII metals selected from platinum, palladium, iridium and ruthenium in elemental or compound form on a support.

Preferred catalysts will employ platinum in elemental or compound form as a Group VIII metal and copper or silver in elemental or compound form as a Group IB metal constituent. More preferably, the Group IB and Group VIII metals will consist of copper or silver (with copper being generally preferred to silver) and platinum in their elemental or compound forms. Especially preferred is a supported bimetallic catalyst of copper and platinum.

The proportions and amounts of the selected Group VIII and Group IB metals in these preferred catalysts may vary depending on the particular chlorinated alkane or alkanes involved, but in general terms, the Group IB metal can be from 0.01 to 20 percent by weight (on an elemental basis) of the catalyst, with a selected Group VIII metal comprising from 0.01 to 5.0 percent by weight (also on an elemental basis) of the catalyst.

More preferably, a selected Group IB metal will be from 0.05 to 15 percent by weight of the catalyst (on an elemental basis) and a selected Group VIII metal will be from 0.03 to 3.0 percent by weight of the catalyst. Most preferably, the Group IB metal can be from 0.1 to 10 percent by weight of the catalyst (on an elemental basis) and the Group VIII metal from 0.05 to 1.0 percent by weight of the catalyst.

The support in each of these catalysts can be any of those conventionally employed in the past, but is preferably silica or carbon, with carbon being more preferred. Preferably the carbon support is a high surface area carbon, for example, a carbon having a specific surface area in an unimpregnated condition of 200 m²/g or more, especially 400 m²/g or more, and most especially 600 m²/g or more. An example of a commercially-available carbon which has generally been found to be well-suited for use in the present invention is a coal-based carbon produced by Calgon Carbon Corporation under the designation "BPLF3", and may generally be characterized as having a specific surface area of 1100 m²/g to 1300 m²/g, a pore volume of 0.7 to 0.85 cm³/g, and an average pore radius of 12.3 to 14 angstroms (1.23 to 1.4 nm). Based on an X-ray fluorescence analysis of this carbon, a typical bulk composition of the BPLF3 carbon has been determined to be as follows (the percents being weight percents): silicon, 1.5 percent; aluminum, 1.4 percent; sulfur, 0.75 percent; iron, 0.48 percent; calcium, 0.17 percent; potassium, 0.086 percent; titanium, 0.059 percent; magnesium, 0.051 percent; chlorine, 0.028 percent; phosphorus, 0.026 percent; vanadium, 0.010 percent; nickel, 0.0036 percent; copper, 0.0035 percent; chromium, 0.0028 percent; and manganese, 0.0018 percent (the remainder being carbon). Other carbons may however be preferable for specific chlorinated alkane to less-chlorinated alkene conversions. For example, either a coconut-based carbon such as produced by Calgon Carbon Corporation under the designation PCB (having a published or advertised specific surface area of from 1150 to 1250 m²/g and a pore volume of 0.72 cm³/g ) or a wood-based carbon such as produced by Calgon Carbon Corp. as WSIV Special carbon (having an advertised specific surface area of 1400 m²/g, and a pore volume of 1.25 cm³/g) is preferred for the conversion of 1,2,3-trichloropropane to propylene because of the lower rate of catalyst deactivation observed with the use of these catalyst supports in this process application as compared to the aforementioned BPLF3 carbon.

The reaction conditions may again vary, depending for example on the particular catalyst and the particular chlorinated alkane feedstock or feedstocks involved, as well as on whether a process is to be conducted in the gas phase or in the liquid phase. In general, however, reaction pressures in the gas phase processes can range from atmospheric up to 1500 psig (10.3 MPa (gauge)), with temperatures of from 100 deg. C. to 350 deg. C., residence times of from 0.25 seconds to 180 seconds, and hydrogen/ chlorinated alkane feed ratios ranging on a molar basis from 0.1:1 to 100:1. More preferably, reaction pressures will range from 5 psig (0.03 MPa (gauge)) to 500 psig (3.4 MPa (gauge)), with temperatures of from 180 deg. C. to 300 deg. C., residence times of from 0.5 seconds to 120 seconds, and hydrogen/chlorinated alkane feed ratios of from 0.5:1 to 20:1. Most preferably, reaction pressures in the gas phase processes will range from 40 psig (0.28 MPa (gauge)) to 300 psig (2.1 MPa (gauge)), with temperatures of from 200 deg. C. to 260 deg. C., residence times of from 1 second to 90 seconds, and hydrogen/chlorinated alkane molar feed ratios of from 0.75:1 to 6:1. It is expected that liquid phase processes (which can be conducted in a batchwise or continuous manner of operation, as desired) will operate at reaction pressures ranging from atmospheric pressure up to 3000 psig (20.6 MPa (gauge)), temperatures of from 25 degrees Celsius to 350 degrees Celsius, residence times ranging from one minute to 30 minutes, and hydrogen to chlorinated alkane molar feed ratios of from 0.1:1 to 100:1.

A particularly preferred application of the present invention is the gaseous-phase reaction of 1,2-dichloropropane or PDC with hydrogen to form reaction products including propylene in a commercially substantial proportion.

For this particular process, the catalyst is preferably a supported bimetallic platinum/copper catalyst comprising from 0.01 to 5.0 percent by weight of platinum (calculated on an elemental basis) and from 0.01 to 15 percent by weight of copper (also calculated on an elemental basis) on a carbon support having a specific surface area of at least 200 m²/g. More preferably, the catalyst includes from 0.10 to 3.0 percent by weight of platinum and from 0.05 to 5 percent by weight of copper, and the carbon support has a specific surface area of at least 500 m²/g. Most preferably, the catalyst includes from 0.20 to 1.0 percent by weight of platinum and from 0.1 to 2.0 percent by weight of copper, and the carbon support has a specific surface area of at least 800 m²/g. A particularly preferred carbon is the BPLF3 carbon described extensively above.

Preferably the catalyst for this process (and for other contemplated chlorinated alkane to less-chlorinated alkene processes which desirably produce propylene over propane) will have been pretreated by exposure to a chloride source, for example, hydrogen chloride, to improve initial selectivity to propylene over propane and to make the product stream immediately useful in an allyl chloride process (wherein propane in the feed can react with chlorine to form 1-chloropropane, which because of a similarity in boiling points to allyl chloride is difficult to remove), for example, or to minimize venting in a propylene oxide process of any propane in the product stream fed or recycled to the propylene oxide process. In this regard, catalysts of the present invention which have been impregnated, dried and reduced under hydrogen have been observed to produce propane with decreasing selectivity and propylene with increasing selectivity over time. The initial chloride source pretreatment substantially reduces, however, the amounts of venting or downstream processing involved in making use of the product stream in a propylene oxide process or allyl chloride process, respectively.

Catalysts that have been pretreated by exposure to a chloride source and not reduced, or which have been merely dried and started up (and which have been in essence treated with hydrogen chloride in situ on startup), have been observed to produce the least amount of propane initially, albeit with a substantial penalty in conversion. For this reason, it is considered that it will generally be preferable to both reduce and pretreat (by exposure to a chloride source) a given catalyst, as opposed to reducing the catalyst solely, or not reducing the catalyst at all in favor of chloride-source pretreatment or treatment with HCl in situ on startup.

It will also be preferred in the context of this process (and further, in each of the various processes contemplated herein) to employ hydrogen chloride in the feed to reduce coking and catalyst deactivation rates, as exemplified below. Preferably the rate of conversion loss is no more than 0.03 percent per hour, and more preferably is no more than 0.01 percent per hour.

The pressure under which the preferred gas phase PDC to propylene reaction is conducted preferably ranges from atmospheric pressure to 1500 psig (10.3 MPa (gauge)), more preferably from 5 psig (0.03 MPa (gauge)) to 500 psig (3.4 MPa (gauge)), and most preferably from 50 psig (0.34 MPa (gauge)) to 300 psig (2.1 MPa (gauge)). The temperature will preferably be from 100 deg. C. to 350 deg. C., more preferably will be from 180 deg. C. to 300 deg. C., and most preferably will be from 200 deg. C. to 260 deg. C.

Preferred residence times will be from 0.25 seconds to 180 seconds, more preferably will be from 1.0 seconds to 20 seconds, and most preferably will be from 5 to 15 seconds. Hydrogen to PDC feed ratios will preferably be (again, on a molar basis) from 0.1:1 to 100:1. More preferably, the hydrogen to PDC feed ratios will be from 0.3:1 to 10:1, and most preferably from 0.5:1 to 3:1. Preferably this process will on a commercial-scale include a recycle stream comprised of unreacted PDC and 2-chloropropane (as a byproduct of the reaction), although as suggested above the amount of 2-chloropropane that is produced and recycled is preferably minimized by a chloride source pretreatment of the bimetallic Pt/Cu catalyst.

### Illustrative Examples

The present invention is more particularly illustrated by the examples which follow hereafter. In Examples 1-9, a number of catalyst preparations were evaluated for converting 1,2-dichloropropane to reaction products including propylene. Several parameters were measured and/or calculated for purposes of comparison, including PDC conversion (100 minus the mol percent of PDC in the test reactor effluent, excluding unreacted hydrogen and the hydrochloric acid produced by the reaction), selectivity to a given component (mols of component divided by mols PDC converted, multiplied by 100), liquid hourly space velocity (in hr⁻¹, the volume of liquid PDC fed per hour to the reactor divided by the packed bed volume of catalyst in the reactor), and catalyst productivity (on a basis of kilograms of propylene produced per hour per cubic meter of catalyst used).

For each example in Examples 1-9, PDC was converted to propylene by flowing hydrogen and PDC in the gas phase over a given catalyst to be evaluated. Liquid PDC was pumped via a piston pump through 1/16th inch (1.6 mm) (O.D.) nickel tubing to a Monel™ alloy (Huntington Alloys, Inco Alloys International, Inc.) gas sample cylinder packed with glass beads (unless specifically noted, all fittings and tubing were of Monel™ alloy). The 1/16th inch (1.6 mm) tubing extended to the center of the sample cylinder, with the sample cylinder being heated to a vaporization temperature of 110 degrees Celsius by electrical heat tracing. A thermocouple was used to monitor the skin temperature of the sample cylinder.

The flow of the hydrogen feed stream was controlled by a pre-calibrated mass flow controller. The desired flow of hydrogen was passed through the heated sample cylinder, where mixing of the gaseous PDC and hydrogen occurred. The mixed gases were then passed into a charged Monel™ tubular reactor (0.75 in. (1.9 cm.) O.D., 18 inches (45.7 cm.) in length) heated by ceramic lined electric elements to a desired reaction temperature.

The catalyst (1.2 cubic centimeters) was in each case charged into the reactor between 3 mm glass beads, and placed in the middle of the reactor. The catalyst was thereafter dried under a flow of nitrogen for one hour at 130 degrees Celsius, and then reduced under a 5:1 molar ratio of flowing nitrogen and hydrogen. In reducing the catalyst, the temperature was ramped up from 130 to 220 degrees Celsius at 3 degrees per minute, and then held at 220 degrees for a total reducing cycle time of about 2 hours.

Upon reaction of the mixed hydrogen and PDC in the reactor at a prescribed reaction temperature, the effluent from the reactor passed to a gas sampling valve, which provided gaseous aliquots for online gas chromatographic analysis in a Hewlett-Packard Model 5890 Series II gas chromatograph (Hewlett-Packard Company). The gas chromatograph was equipped with a flame ionization detector, and used 30 meter by 0.53 millimeter (I.D.) 100 percent methyl silicone/fused silica and 30 meter by 0.53 millimeter (I.D.) porous polymer-lined fused silica columns to separate the various reaction products. Response factors were conventionally determined by injections of gravimetrically-prepared standards of the individual reaction products. These response factors were applied in conjunction with individual peak areas and the total mols of all reaction products to determine the mol percents of individual components in the reactor effluent, and the selectivity to individual reaction products as described above.

### Example 1

For this example a platinum-copper catalyst was prepared on a carbon support for comparison to platinum on a carbon support alone, to copper on a carbon support alone, and to the carbon support alone.

For the platinum-copper catalyst, an aqueous H₂PtCl₆ stock solution was prepared by dissolving 3.179 grams of H₂PtCl₆·6H₂O (J. T. Baker, Inc.; Baker Analyzed Grade, 37.6 percent Pt) in 100.00 mL of deionized and distilled water. 0.381 grams of CuCl₂ (Aldrich Chemical Company, Inc., 99.999 percent purity) were placed in a 250 mL Erlenmeyer flask, and 8.305 grams of the H₂PtCl₆ stock solution were added with swirling to dissolve the CuCl₂. The solution was then diluted with 42.64 grams of deionized, distilled water and swirled. 40.04 grams of Calgon BPLF3 activated carbon (6 x 16 mesh (3.4 mm x 1.2 mm), Calgon Carbon Corp., Pittsburgh, Pa.) were added to the flask, and the flask was agitated rapidly so that the carbon carrier was evenly coated with the aqueous Pt/Cu solution. The catalyst preparation was dried in an evaporating dish in air at ambient temperatures for 18 hours, and then further dried in an oven in air at 120 degrees Celsius for 2 hours before being charged to the reactor and reduced. The resulting catalyst was comprised of 0.25 percent by weight of platinum (on an elemental basis) and 0.45 percent by weight of copper (also on an elemental basis).

Similar methods of preparation were used to make catalysts of 0.48 percent by weight of copper on the Calgon BPLF3 activated carbon support (omitting the chloroplatinic acid solution), and of 0.25 percent by weight of platinum on the same activated carbon (omitting the CuCl₂ from the process).

Reactions were run at various reaction temperatures on the apparatus described above with separate charges of each of these catalysts, as well as running reactions at various temperatures with an unimpregnated Calgon BPLF3 activated carbon support as a test for reactivity of the support.

Each of the catalysts was loaded into the 18 inch (45.7 cm) long tubular reactor described above, using about 1.2 cubic centimeters of catalyst. The liquid hourly space velocity (LHSV) was 1.25, and the hydrogen and PDC were fed to the reactor at a molar ratio of 6.0:1. Reactor temperatures, PDC conversions, productivities, and individual product selectivities for the runs with these catalysts are shown as follows in Table 1, in which the propylene yields for the runs with the bimetallic platinum/copper catalyst were 30.1 percent (at 220 degrees, or (30.64 x 98.16)/100), and 67.4 percent (at 250 degrees Celsius, or (70.53 x 95.5)/100):

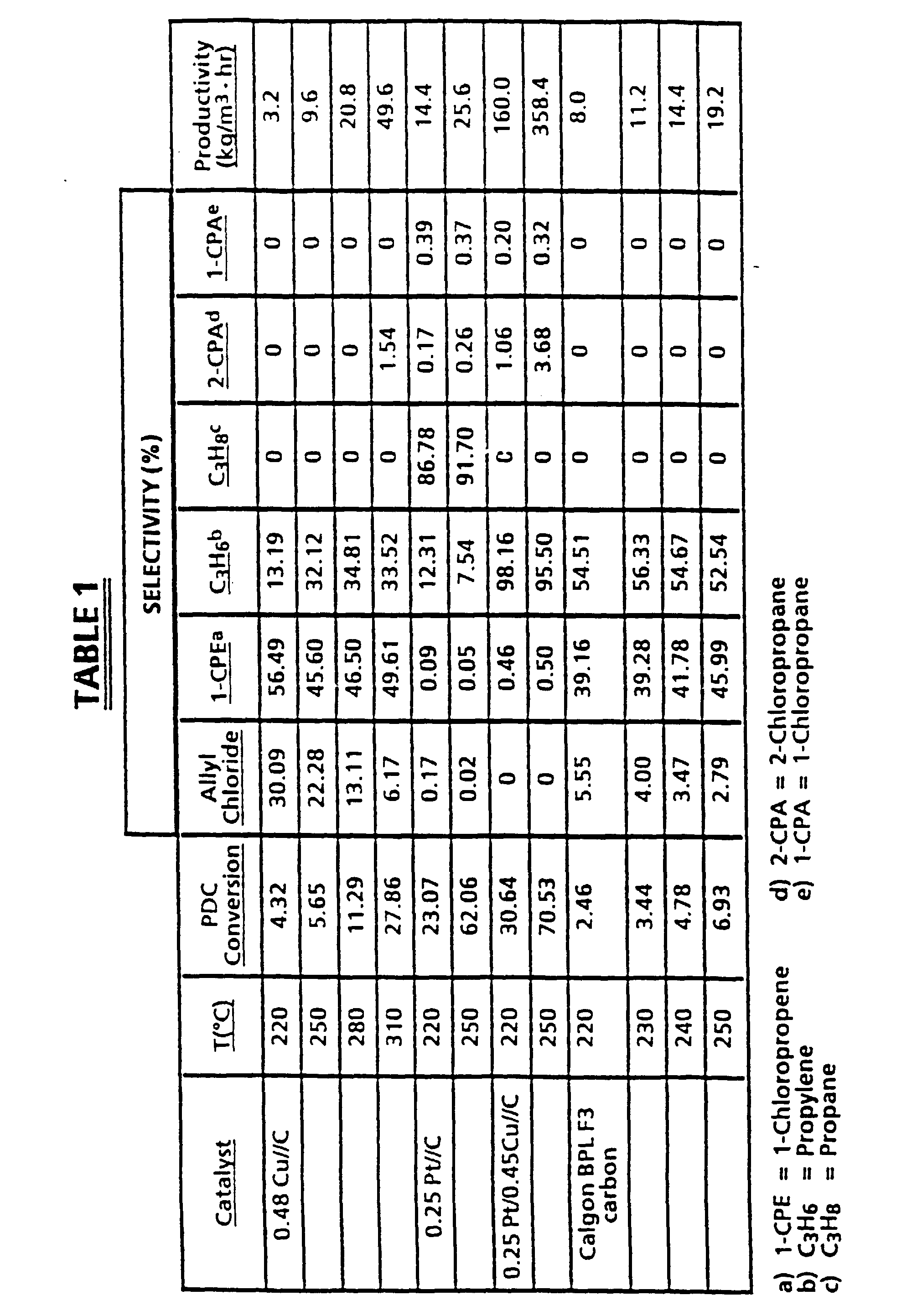

### Examples 2-6

In Examples 2-6, platinum-copper catalysts were prepared which used the same activated carbon support as in Example 1. The catalysts were each prepared, charged and reduced as in Example 1, except that the catalysts for these examples used different amounts of platinum and copper (both on an elemental basis).

In the runs with each of these catalysts, 1.2 cubic centimeter charges of a given catalyst were employed in the 18 inch (45.7 cm) Monel™ reactor, and the hydrogen to PDC feed ratio on a molar basis was set at 5:1 (rather than the 6:1 ratio seen in Example 1). Liquid hourly space velocities and reaction temperatures were varied. The results of these runs are shown in Table 2:

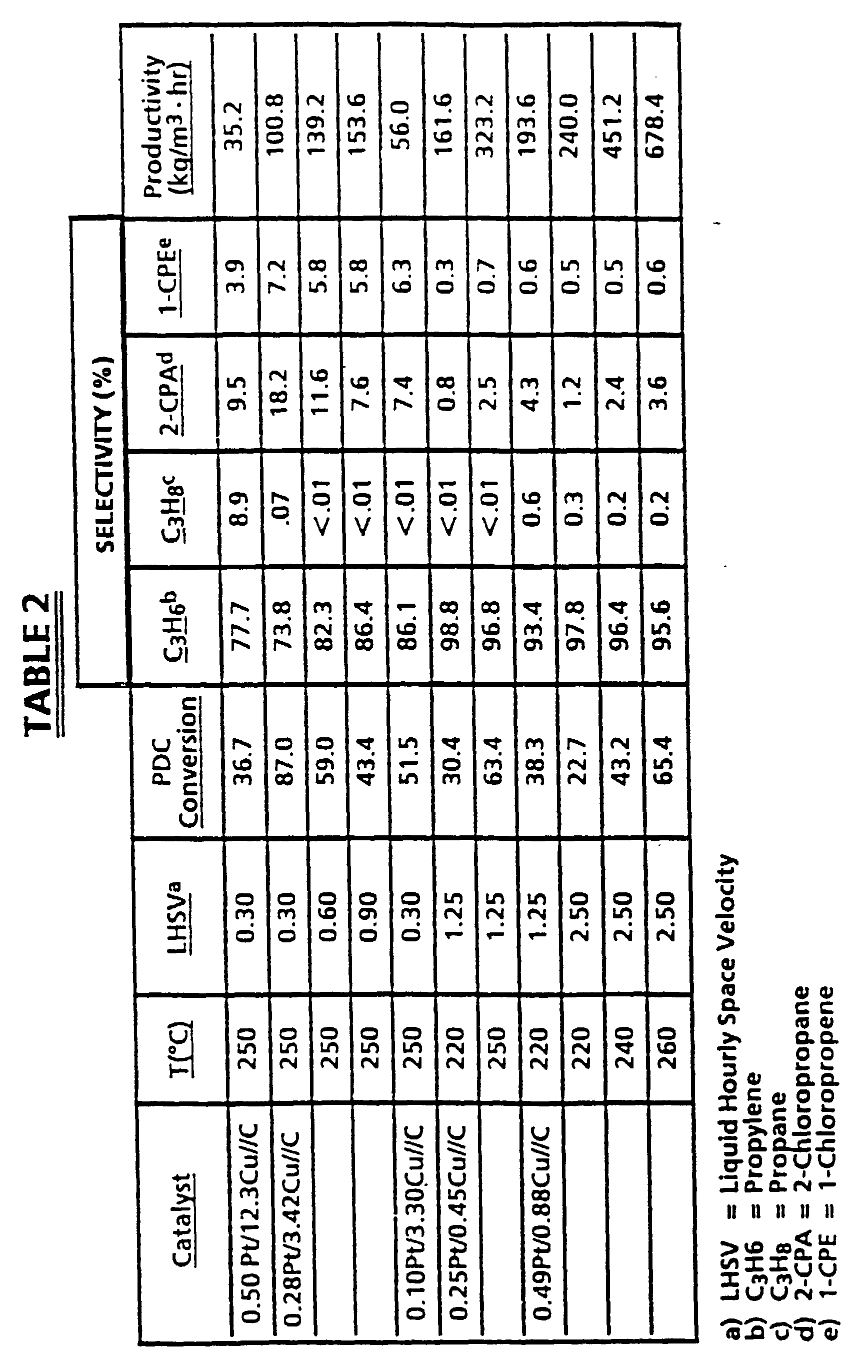

### Example 7

The catalyst prepared for this Example included palladium instead of platinum, along with the copper of previous examples.

In making the palladium-copper catalyst of this example, 0.393 grams of PdCl₂ (Aldrich Chemical Company, Inc., 99.999% purity) was dissolved in 2 mL of 12M hydrochloric acid (HCl) and diluted to 25.00 mL in a volumetric flask with distilled, deionized water. 0.097 grams of CuCl₂ (Aldrich Chemical Company, Inc., 99.999% purity) was placed in a 50 mL Erlenmeyer flask, and 1.435 grams of the prepared H₂PdCl₄ stock solution was added with swirling to the flask containing the CuCl₂. When the CuCl₂ had dissolved, the resulting solution was diluted with 11.00 grams of deionized, distilled water and swirled.

10.00 grams of Calgon BPLF3 activated carbon (6 x 16 mesh (3.4 mm x 1.2 mm), Calgon Carbon Corp.) were then added with agitation to coat the carbon evenly with solution, and the catalyst thus prepared was dried in air at ambient temperatures for 18 hours. The catalyst, which comprised 0.13 percent by weight of palladium on an elemental basis and 0.45 percent by weight of copper on an elemental basis on the activated carbon support, was then further air-dried in an oven at 120 degrees Celsius for 2 hours and reduced as described previously.

Three runs were conducted sequentially with this catalyst in the apparatus and according to the general procedure set forth just prior to Example 1 above, using a single 1.2 cubic centimeter charge of catalyst in the tubular reactor. The results from these runs are shown in Table 3 below, and suggest or show that a palladium/copper catalyst is considerably less active than the previous platinum/copper catalyst (0.25 Pt/0.45 Cu//C) of equimolar proportions for the conversion of PDC to propylene.

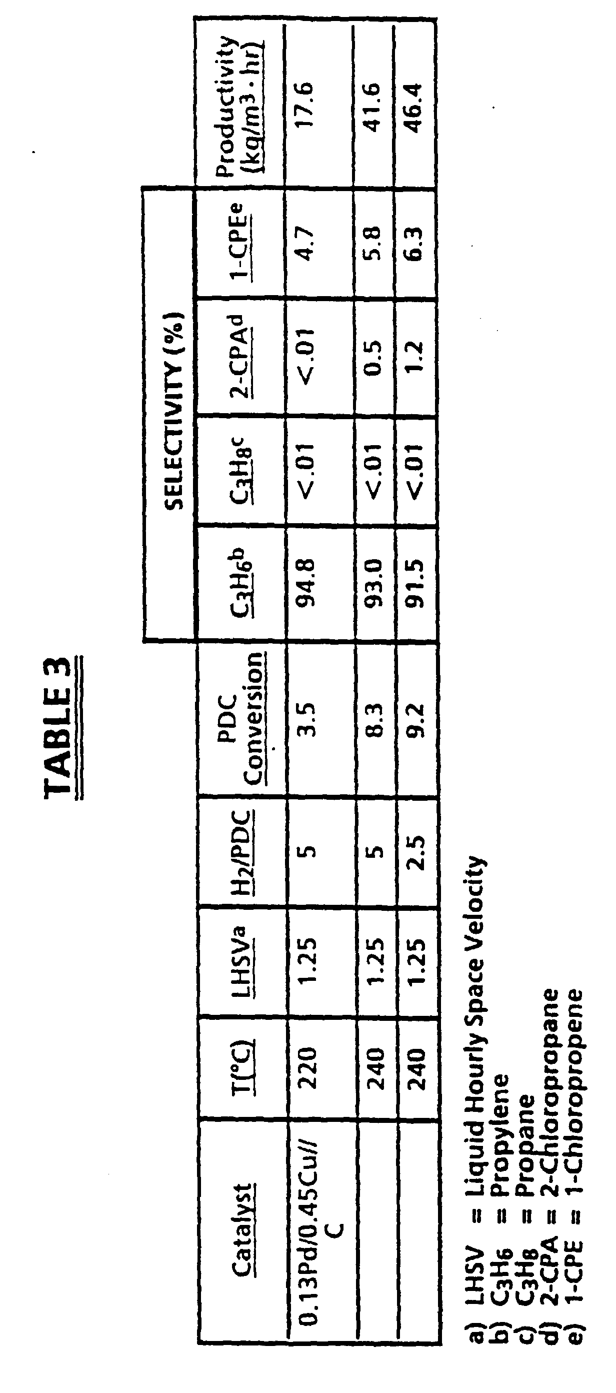

### Example 8

The catalyst for the sample runs in this example was comprised of 0.25 percent by weight of iridium (on an elemental basis) and 0.45 percent by weight of copper (again, on an elemental basis) on the same activated carbon support used in previous examples.

The iridium-copper catalyst was prepared via a stock solution of IrCl₄, by dissolving 0.911 grams of IrCl₄·xH₂O (53.4 percent iridium) in 4.988 grams of 0.1 M HCl solution in a 50.00 mL volumetric flask, and diluting this solution to 50.00 mL with deionized, distilled water.

0.095 grams of CuCl₂ (Aldrich Chemical Company, Inc., 99.999% purity) were placed in a 50 mL Erlenmeyer flask, and 2.553 grams of the IrCl₄ stock solution were added with swirling to dissolve the CuCl₂. This solution was diluted with 10.00 grams of deionized, distilled water. 10.00 grams of Calgon BPLF3 activated carbon (6 x 16 mesh; 3.4 mm x 1.2 mm) were added to the flask, and the flask agitated thoroughly to ensure that the activated carbon was evenly coated with the aqueous solution of IrCl₄ and CuCl₂. The catalyst made in this fashion, with 0.25 percent by weight of iridium (elemental basis) and 0.45 percent by weight of copper (elemental basis) on activated carbon, was air-dried as in previous examples, that is, at ambient temperature for 18 hours and then in an oven at 120 degrees Celsius for another 2 hours, and then reduced.

Three runs were conducted with the catalyst in the apparatus and according to the procedures described previously, with 1.2 cubic centimeter charges of catalyst being charged to the reactor for each set of reaction conditions. The remaining reaction parameters and the results of the runs are as indicated in Table 4 below.

These results suggest or show that the iridium/copper catalyst is generally less selective for converting PDC to propylene than a like-proportioned platinum/copper catalyst.

### Example 9

The catalyst tested in this example comprised 0.29 percent by weight on an elemental basis of platinum and 0.75 percent by weight of silver on the Calgon activated carbon, and was prepared first by making an aqueous stock solution from 0.048 grams of Pt(NH₃)₄(NO₃)₂, 0.116 grams of silver nitrate, and 12.43 grams of distilled, deionized water in a 50 mL Erlenmeyer flask. The same activated carbon as used in previous examples was then added (at 9.68 grams) to the flask containing the stock solution, and the flask agitated and the catalyst dried as in previous examples. A single 1.2 cubic centimeter charge (weighing 500.1 milligrams) of the catalyst was placed in the reactor and reduced, then tests were conducted sequentially with this catalyst at several hydrogen to PDC molar feed ratios.

The results from the testing are shown in Table 5 below.

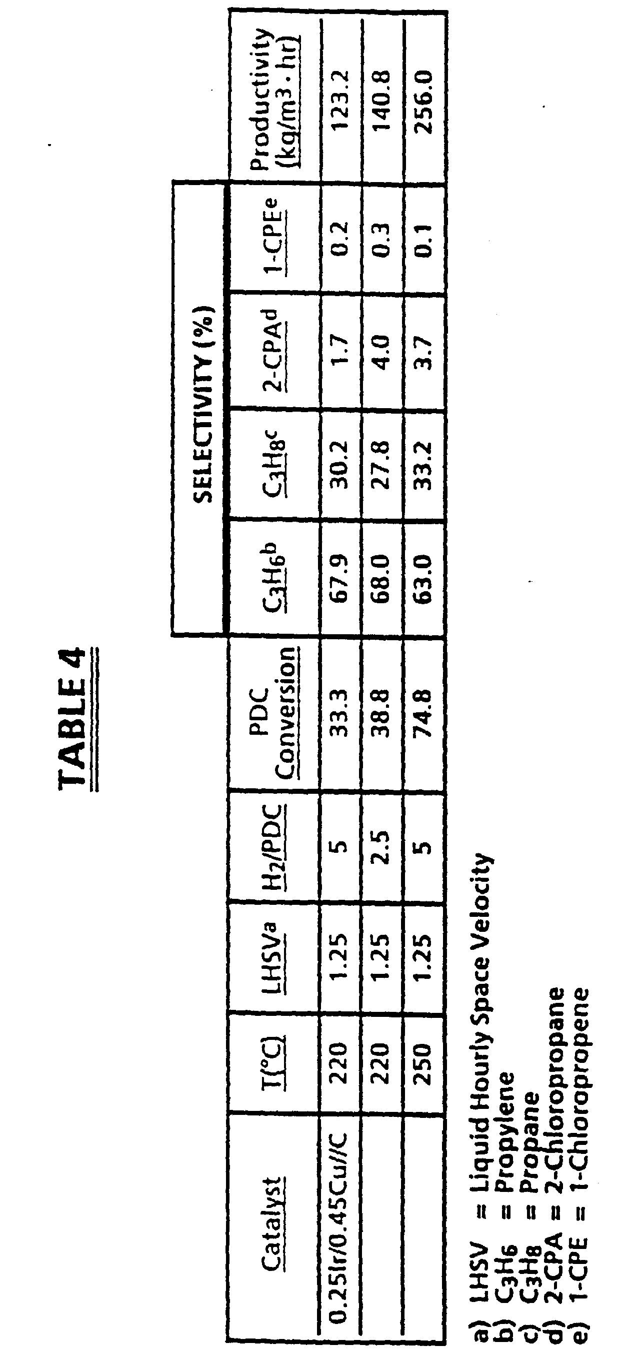

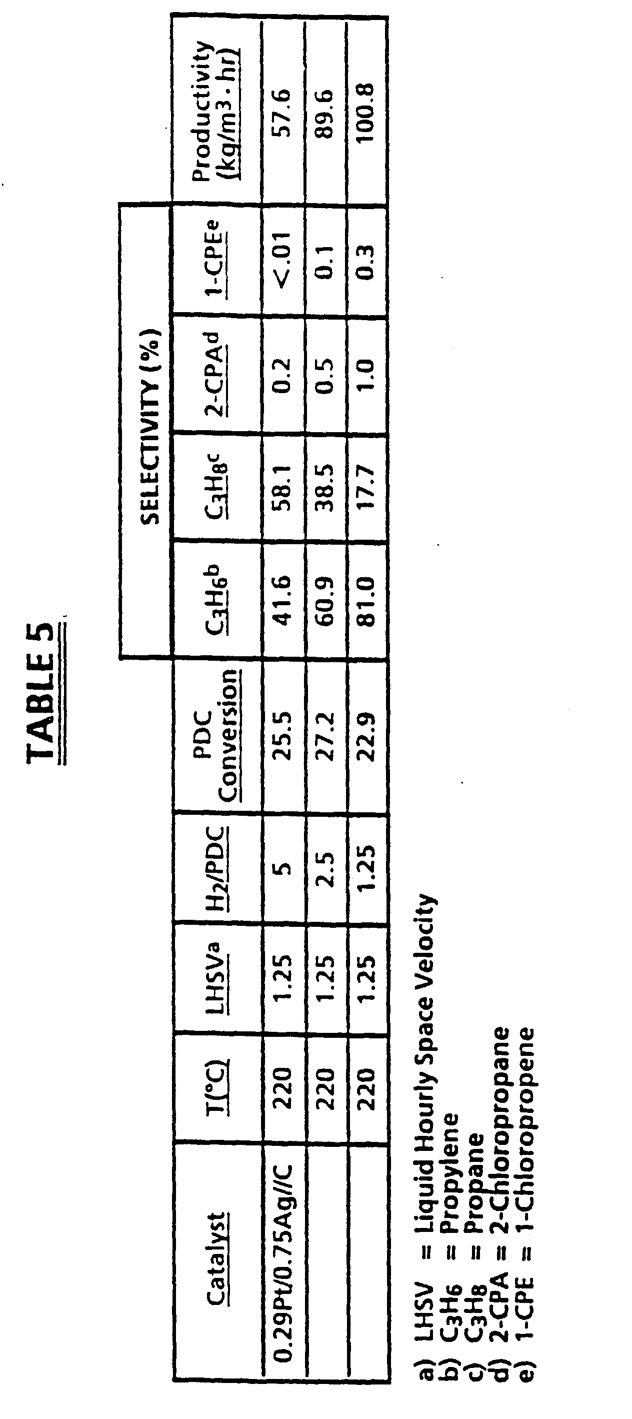

### Examples 10-12

Examples 10-12 below focus on the reaction with hydrogen of various chlorinated alkane feedstocks over a supported platinum/copper catalyst of the present invention, with the catalyst having been prepared according to the method of Example 1 above.

In each instance, the particular feedstock to be reacted was pumped via a high pressure syringe pump through 1/16 inch (O.D.) (1.6 mm) Monel™ nickel alloy tubing (unless specifically noted below all of the components, tubing and fittings of the test reactor apparatus were also made of Monel™ nickel alloy (Huntington Alloys, Inco Alloys International, Inc.)) into a packed sample cylinder serving as a feed evaporator.

The 1/16th inch (1.6 mm) tubing extended almost to the center of the packed cylinder, which was heated to a vaporizing temperature of 180 degrees Celsius using electrical heat tracing. Vaporization of the feedstock was accomplished in the feed line, so that the feedstock was superheated when combined with the hydrogen feed stream. Thermocouples were used to monitor the skin temperature of the feed evaporator and the temperature of the gas exiting the feed evaporator, and the temperature of the feed evaporator was computer controlled.

The hydrogen feed stream was metered to a preheater using a Model 8249 linear mass flow controller from Matheson Gas Products, Inc. Secaucus, N.J., with the preheater consisting of a packed sample cylinder wrapped with electrical heat tracing. Thermocouples were used to monitor both the skin temperature of the preheater and the temperature of the gas exiting the preheater. The preheater temperature was set at 140 degrees Celsius.

Vaporized feedstock exiting the evaporator was mixed with the hydrogen gas from the preheater in a 2 foot (0.61 meter) long section of 1/4 inch (0.64 cm) tubing maintained at a temperature of 140 degrees Celsius. The mixed gases then were passed into and reacted within a tubular reactor (1/2 inch (1.27 cm) O.D., 12 inches (30.5 cm) in length) located within an aluminum block heated by a cartridge heater and controlled via a computer.

The catalyst charge (0.6 grams in each of Examples 10-12) was generally placed in the tubular reactor over a glass wool support contained in the center of the reactor tubing. The catalyst was then covered with a plug of glass wool.

The charged catalyst was dried for from 8 to 24 hours at 150 degrees Celsius under a nitrogen purge. The catalyst was thereafter reduced by passing hydrogen through the reactor at a flow rate of 34 mL/minute for 24 hours, and the reactor temperature was then lowered to the temperature setpoint of the particular catalyst run. The reactor temperature and hydrogen gas flow were allowed to equilibrate for about 1 hour before the liquid feedstock flow was started into the apparatus.

After reacting the feedstock and hydrogen in the vapor phase in the tubular reactor thus prepared, the products from the reaction were passed to a gas sampling valve, which provided gaseous aliquots for online gas chromatographic analysis in a Hewlett-Packard Model 5890 Series II gas chromatograph (Hewlett-Packard Company). The gas chromatograph was equipped with a flame ionization detector, and used 30 meter by 0.53 millimeter (I.D.) 100 percent methyl silicone/fused silica and 30 meter by 0.53 millimeter (I.D.) porous polymer-lined fused silica columns to separate the various reaction products. Response factors were conventionally determined by injections of gravimetrically-prepared standards of the individual reaction products. These response factors were applied in conjunction with individual peak areas and the total mols of all reaction products to determine the mol percents of individual components in the reactor effluent, and the selectivity to individual reaction products.

The feedstocks, conditions and results of Examples 10-12 are reported in Table 6 below:

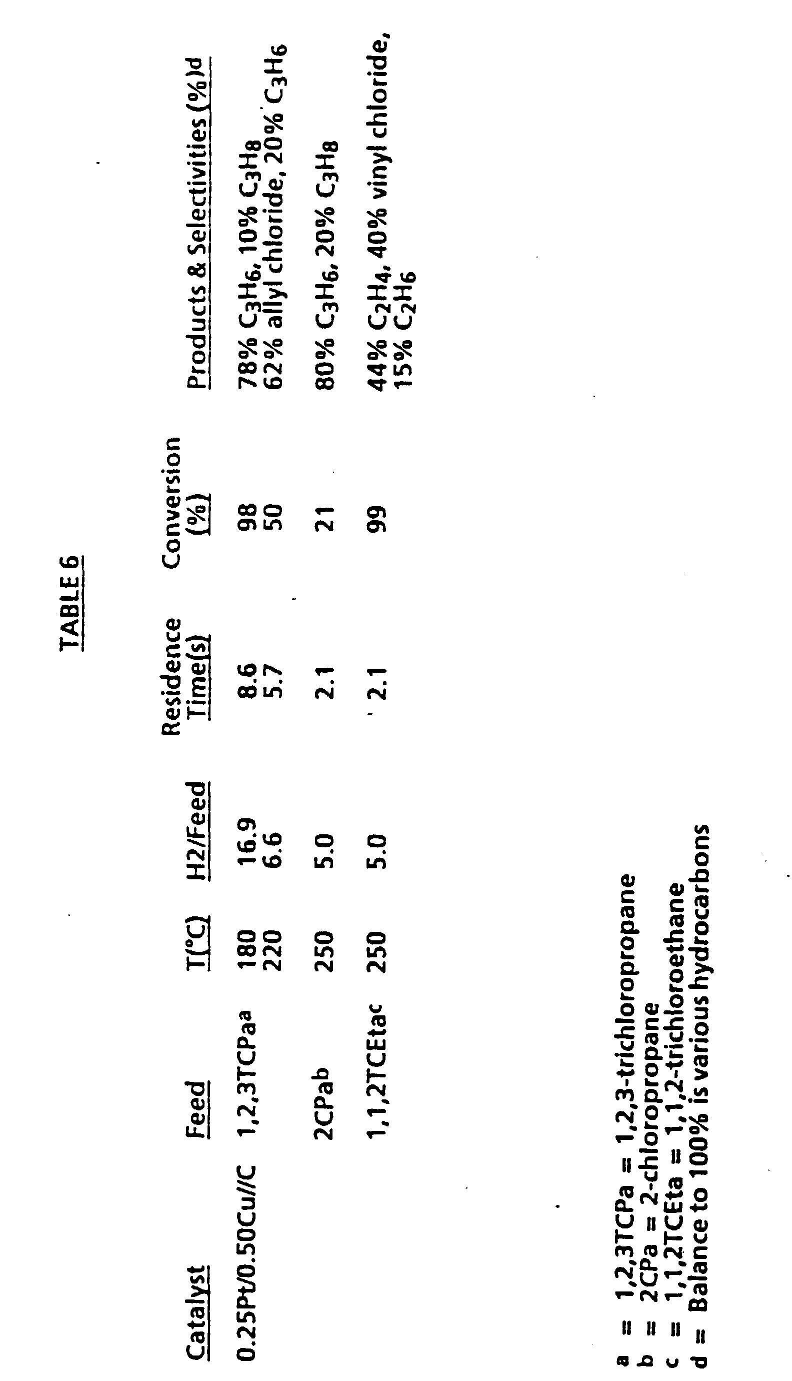

### Examples 13-17

The experimental method and apparatus of Examples 10-12 above were employed (except as indicated below) in these Examples for determining the effects on the rate of catalyst deactivation in converting 1,2,3-trichloropropane to propylene of adding hydrogen chloride (HCl) to a 1,2,3-trichloropropane feed , and of omitting HCl from the feed but using a different wood- or coconut-based carbon support.

Bimetallic platinum/copper catalysts were prepared for these examples in the ratios indicated in Table 7, by dissolving H₂PtCl₆·6H₂O (J. T. Baker, Inc.; Baker Analyzed Grade, 37.6 percent Pt) in deionized and distilled water. An amount of CuCl₂ (Aldrich Chemical Company, Inc., 99.999 percent purity) was placed in a 250 mL Erlenmeyer flask, and the H₂PtCl₆ stock solution was added with swirling to dissolve the CuCl₂. The solution was then diluted with deionized, distilled water and swirled. An activated carbon (Calgon BPLF3 carbon, 6 x 16 mesh (3.4 mm x 1.2 mm), Calgon Carbon Corp., Pittsburgh, Pa. for the runs with HCl in the feed, Calgon PCB brand coconut-based activated carbon or Calgon WSIV Special wood-based activated carbon) was added to the flask, and the flask was agitated rapidly so that the carbon carrier was evenly coated with the aqueous Pt/Cu solution.

The various catalysts were charged to the reactor and dried under flowing nitrogen at 90 cubic centimeters per minute, at temperatures increasing from 25 degrees Celsius to 120 degrees Celsius at a rate of 3 degrees Celsius per minute with the 120 degree temperature being held for an hour. These catalysts were then reduced with flowing hydrogen at 90 cubic centimeters per minute, with the temperature being raised from 120 degrees Celsius to 220 degrees Celsius at 3 degrees per minute and the 220 degree temperature being held for 2 hours.

The reaction temperature was set at the same 220 degrees Celsius, with a reaction pressure of 0.14 MPa (gauge) (20 pounds per square inch, gauge), a residence time of 3.5 seconds, a liquid hourly space velocity of 0.91 hr⁻¹ and a hydrogen to 1,2,3-trichloropropane (TCP) molar feed ratio of 6.0 to 1. Hydrogen chloride when added was metered in as a gas in a 3.0 to 1 ratio with respect to the 1,2,3-trichloropropane.

Selectivities to allyl chloride and to propylene, as well as percent conversion of the 1,2,3-trichloropropane, were assessed initially for all of the catalysts, and at a later period in time when the percent conversion reached 20 percent for the standard BPLF3 carbon-based catalyst as well as for the runs of the BPLF3 carbon-based catalyst involving HCl in the feed. The results are as indicated in Table 7:

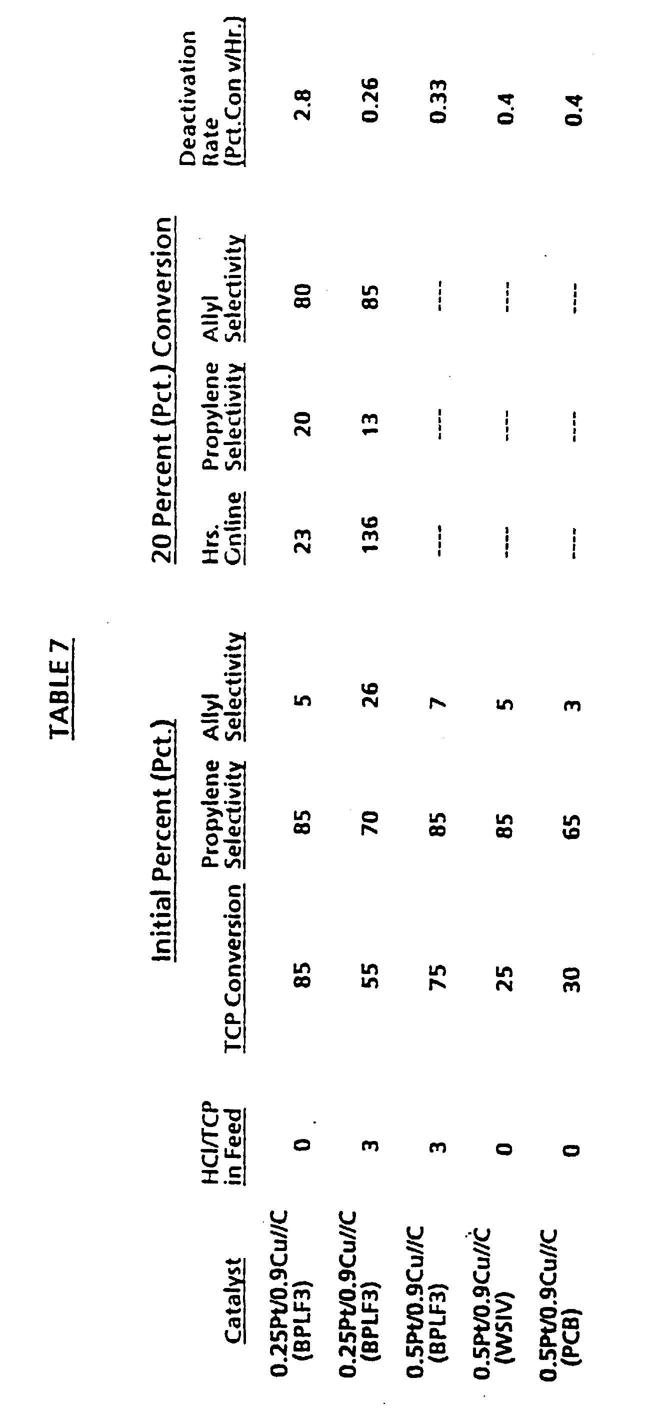

### Examples 18-21

These examples compare the effects on conversion and the selectivities to propylene and propane in a PDC to propylene process of pretreating a bimetallic platinum/ copper catalyst by exposure to a chloride source (hydrogen chloride) without any reduction of the catalyst under hydrogen, of combining reduction under hydrogen with chloride source pretreatment, of using reduction without any chloride source pretreatment, and of simply starting up the dried catalyst without prior exposure to a chloride source or reduction. A quantity of catalyst was prepared according to the methods described in Examples 13-17 above, including 0.5 percent by weight of platinum and 0.9 percent by weight of copper on the Calgon BPLF3 carbon support.

Using the apparatus and basic procedures of Examples 10-12 and 13-17, a first catalyst sample was charged to the reactor and dried under flowing nitrogen at 90 cubic centimeters per minute, at temperatures increasing from 25 degrees Celsius to 120 degrees Celsius at a rate of 3 degrees Celsius per minute and with the 120 degree temperature being held for an hour. The catalyst was then reduced with flowing hydrogen at 90 cubic centimeters per minute, with the temperature being raised from 120 degrees Celsius to 235 degrees Celsius (instead of 220 degrees Celsius, as in Examples 13-17) at 3 degrees per minute and the 235 degree temperature being held for 2 hours. The catalyst prepared and treated in this fashion is catalyst "A" in Table 8 below.

A second sample was charged and dried from 25 degrees Celsius to 120 degrees Celsius under flowing nitrogen, as with the first sample. After holding at 120 degrees for one hour, however, a 50 cubic centimeter per minute flow of hydrogen chloride was introduced and maintained for one half hour. The temperature was then raised from 120 degrees to 235 degrees Celsius at 3 degrees per minute, under a flow of 140 cc/min of a 2:1 mixture of hydrogen and hydrogen chloride. The resulting chloride source-pretreated catalyst was held at 235 degrees for two hours, and is designated as catalyst "B" in Table 8.

A third catalyst sample was dried from 25 degrees Celsius to 235 degrees Celsius at a rise of 3 degrees Celsius per minute, under flowing nitrogen at 90 cc/min. After holding for 4 hours at 235 degrees Celsius, a flow of 50 cc/min. of pure HCl was begun and maintained over an additional 4 hours. The catalyst "C" in Table 8 corresponds to this third catalyst sample.

Finally, a fourth catalyst sample was held under flowing nitrogen (at 90 cc/min.) as the temperature was raised from 25 degrees Celsius to 235 degrees Celsius at 3 degrees per minute, and then maintained at 235 degrees for 3 hours. This catalyst is catalyst "D" in Table 8.

Reaction conditions for catalysts "A" and "B" were 235 degrees Celsius, 75 psig (0.52 MPa (gauge)), a liquid hourly space velocity of 0.44 hr⁻¹, a residence time of 5 seconds and a hydrogen to PDC molar feed ratio of 3.0. Reaction conditions for catalysts "C" and "D" were somewhat different, in that the residence time was 10 seconds instead of 5 seconds and the hydrogen to PDC ratio was 1:1 instead of 3:1.

The selectivities to propylene, 2-chloropropane (2-CPa) and propane, as well as the percent of PDC converted, are as summarized in Table 8 for each of the catalysts A-D.

**Table 8**

| Catalyst | PDC Conversion (Pct.) | C₃H₈ Selectivity (Pct.) | C₃H₆ Selectivity (Pct.) | 2-CPa Selectivity (Pct.) |
|---|---|---|---|---|
| A | 68 | 48 | 40 | 12 |
| B | 67 | 2 | 86 | 12 |
| C | 26 | 0.25 | 84 | 13 |
| D | 32 | 0.42 | 86 | 11 |

### Example 22

The feedstock for this run was 1,1,2-trichloroethane. A 0.5 percent by weight of platinum, 0.9 percent by weight of copper on BPLF3 carbon catalyst was prepared, charged, dried, and reduced for use in this Example as in previous Examples. Using the apparatus of Examples 10-12, 1,1,2-trichloroethane was fed with hydrogen to the reactor in a 1:1 molar feed ratio and with a 5.0 second residence time, at 76 psig (0.52 MPa (gauge)) and 235 degrees Celsius. Eighty (80) percent of the 1,1,2-trichloroethane was observed to be converted initially to reaction products including vinyl chloride at 76 percent selectivity, ethylene at 14 percent selectivity, and ethane at 8 percent selectivity.

A second run was then conducted at a 3:1 molar feed ratio of hydrogen to 1,1,2-trichloroethane (other conditions being the same), with an initial conversion rate of 84 percent. Products were vinyl chloride at 86 percent selectivity, ethylene at 8 percent selectivity and ethane at 5.5 percent selectivity.

### Example 23

The apparatus and procedures of Example 22 were used with a feedstock of 1,1,1,2-tetrachloroethane, except that the molar feed ratio of hydrogen to 1,1,1,2-tetrachloroethane was set at 1.6:1 and the residence time was 4.0 seconds instead of 5.0. Virtually all of the 1,1,1,2-tetrachloroethane (99.95 percent) was initially converted to reaction products including vinylidene chloride at 93 percent selectivity, vinyl chloride at 1 percent selectivity, trichloroethylene at 3 percent, and ethane and ethylene at 3 percent combined. Catalytic activity declined fairly rapidly thereafter due (it is believed) to polymerization of the vinylidene chloride on the catalyst.

### Examples 24-33

The feedstock for these Examples was 2-chloropropane, with propylene the desired product. A bimetallic platinum/copper on carbon catalyst was prepared by initially dissolving H₂PtCl₆·6H₂O (J. T. Baker, Inc., Baker Analyzed Grade, 37.6 percent Pt) in distilled, deionized water. A portion of this stock solution was added with swirling to a 50 mL Erlenmeyer flask containing a corresponding amount of CuCl₂ (Aldrich Chemical Co., Inc., 99.999 percent purity) until the CuCl₂ was dissolved. The solution was diluted with additional distilled, deionized water, and then Calgon's BPLF3 activated carbon was added to the flask with sufficient agitation to coat the carbon fully with the solution, and to form a catalyst which after drying and being reduced comprised 0.9 percent by weight of copper and 0.5 percent by weight of platinum.

The catalyst was air-dried, charged and reduced as in previous examples. The apparatus and procedures of Examples 10-12 were again employed to carry out a series of runs with 2-chloropropane at a constant pressure of 76 psig (0.52 MPa (gauge)), but at varying residence times (see Table 9 below), varying hydrogen to 2-chloropropane molar feed ratios (Table 10) or varying temperatures (Table 11).

Analyses of the reaction products from these runs were carried out as in Examples 10-12, with reaction conditions and results shown in Tables 9-11, where "RT" is the residence time, "PE Sel." is the selectivity to propylene, and "PA Sel." is the selectivity to propane:

**Table 9**

| Temp. (°C) | RT (sec) | Feed Ratio | Pct. Conv. | PE Sel. (%) | PA Sel. (%) |
|---|---|---|---|---|---|
| 235 | 3.4 | 2.8 | 18.1 | 89.94 | 9.46 |
| 235 | 4.9 | 2.8 | 22.8 | 89.84 | 9.67 |
| 235 | 6.9 | 2.8 | 29.5 | 89.53 | 10.03 |

**Table 10**

| Temp. (°C) | RT (sec) | Feed Ratio | Pct. Conv. | PE Sel. (%) | PA Sel. (%) |
|---|---|---|---|---|---|
| 235 | 4.9 | 1.4 | 15.4 | 90.16 | 9.16 |
| 235 | 4.9 | 2.8 | 22.8 | 89.84 | 9.67 |
| 235 | 4.9 | 5.6 | 31.7 | 89.57 | 10.06 |

**Table 11**

| Temp. (°C) | RT (sec) | Feed Ratio | Pct. Conv. | PE Sel. (%) | PA Sel. (%) |
|---|---|---|---|---|---|
| 235 | 4.9 | 2.8 | 22.8 | 89.84 | 9.67 |
| 250 | 4.9 | 2.8 | 38.1 | 89.34 | 10.20 |
| 260 | 4.9 | 2.8 | 49.0 | 88.81 | 10.70 |

### Examples 34-39

The procedures and apparatus described in Examples 10-12 were employed with a catalyst (prepared generally as described in previous examples) which was 0.5 percent by weight of platinum, and 0.45 percent by weight of copper on the BPLF3 carbon. The results of these runs (at residence times in the range of 4.6 to 5.6 seconds, at a pressure of 76 psig (0.52 MPa (gauge)), and at various temperature and hydrogen to 2-chloropropane molar feed ratios) are as shown in Table 12.

**Table 12**

| Temp. (°C) | Feed Ratio | Pct. Conv. | PE Sel. (%) | PA Sel. (%) |
|---|---|---|---|---|
| 235 | 5.4 | 31.3 | 67.2 | 32.4 |
| 250 | 5.4 | 57.4 | 49.3 | 50.3 |
| 250 | 2.8 | 36.3 | 64.5 | 35.0 |
| 260 | 2.8 | 50.3 | 62.4 | 37.1 |
| 270 | 2.8 | 65.4 | 61.7 | 37.8 |
| 280 | 2.8 | 77.9 | 63.8 | 35.7 |

### Examples 40-42

For these examples, the same apparatus and procedures were generally employed as in Examples 10-12 above to evaluate and compare several different catalyst combinations for converting PDC to propylene. Each of these catalysts was prepared from a solution in water of the chlorides of the metals involved, and using the Calgon BPLF3 activated carbon. The catalysts were each charged, air- and oven-dried, and reduced in the manner of Examples 1-9.

The reaction temperature for this comparison was 220 degrees Celsius, the pressure was atmospheric, the residence time was 1 second, and the molar feed ratio of hydrogen to PDC was set at 3:1.

The percent conversions and the selectivities to various products (in percent) from these runs are provided in Table 13, wherein "2-CPA" is 2-chloropropane:

**Table 13**

| Catalyst | Conversion | C₃H₆ | C₃H₈ | 2-CPA |
|---|---|---|---|---|
| 0.25Pt/0.13Ru/0.9Cu | 37 | 98 | 1 | 1 |
| 0.26Ru/0.9Cu | 12 | 95 | 3 | 1 |
| 0.5Pd/1.6Cu | 24 | 95 | 1 | 4 |

While various embodiments of the processes and catalysts of the present invention have been described and/or exemplified herein, those skilled in the art will readily appreciate that numerous changes can be made thereto which are nevertheless properly considered to be within the scope of the present invention as defined by the claims below.

## Claims

1. A process for the conversion of a chlorinated alkane feedstock to reaction products including a less chlorinated alkene by reaction with hydrogen in the presence of a supported catalyst characterised in that the catalyst consists of one or more Group IB metals in elemental or compound form and one or more Group VIII metals selected from platinium, palladium, iridium and rutheninium in elemental or compound form on the support.

2. A process as claimed in Claim 1, wherein hydrogen chloride is incorporated in the feed to the process.

3. A process as claimed in Claim 1 or Claim 2, wherein the one or more Group IB metals includes copper.

4. A process as claimed in Claim 1 or Claim 2, wherein the one or more Group IB metals includes silver.

5. A process as claimed in Claim 1 or Claim 2, wherein the one or more Group VIII metals includes palladium.

6. A process as claimed in Claim 1 or Claim 2, wherein the one or more Group VIII metals includes platinum.

7. A process as claimed in Claim 1 or Claim 2, wherein the one or more Group IB metals includes copper or silver and wherein the one or more Group VIII metals includes platinum.

8. A process as claimed in Claim 7, wherein the Group IB and Group VIII metals consist of copper and platinum.

9. A process as claimed in any one of the preceding claims, wherein the catalyst includes from 0.01 to 5.0 percent by weight of Group VIII metal or compound on an elemental basis and from 0.01 to 20 percent by weight of Group IB metal or compound, also on an elemental basis.

10. A process as claimed in Claim 9, wherein the catalyst includes from 0.03 to 3.0 percent by weight of Group VIII metal or compound on an elemental basis and from 0.05 to 15 percent by weight of Group IB metal or compound, also on an elemental basis.

11. A process as claimed in Claim 10, wherein the catalyst comprises from 0.05 to 1.0 percent by weight of Group VIII metal or compound on an elemental basis and from 0.1 to 10 percent by weight of Group IB metal or compound, also on an elemental basis.

12. A process as claimed in any one of the preceding claims, wherein the catalyst support is a carbon having a specific surface area of at least 200 m²/g.

13. A process as claimed in Claim 12, wherein the catalyst support is a carbon having a specific surface area of at least 400 m²/g.

14. A process as claimed in Claim 13, wherein the catalyst support is a carbon having a specific surface are of at least 600 m²/g.

15. A process as claimed in any one of the preceding claims, wherein the feedstock is selected from 2-chloropropane, 1,2-dichloropropane and 1,2,3-trichloropropane and mixtures of these.

## Patentansprüche

1. Verfahren zur Umsetzung eines chlorierten Alkan-Einsatzmaterials zu Reaktionsprodukten, die ein niedriger chloriertes Alken enthalten, durch Reaktion mit Wasserstoff in der Gegenwart eines Katalysators auf einem Träger, dadurch gekennzeichnet, daß der Katalysator aus einem oder mehreren Gruppe IB Metallen in elementarer Form oder Verbindungsform und einem oder mehreren Gruppe VIII Metallen, ausgewählt aus Platin, Palladium, Iridium und Ruthenium, in elementarer Form oder Verbindungsform auf dem Träger besteht.

2. Verfahren nach Anspruch 1, wobei der Zufuhr zu dem Verfahren Chlorwasserstoff zugesetzt wird.

3. Verfahren nach Anspruch 1 oder Anspruch 2, wobei das oder die Gruppe IB Metalle Kupfer umfassen.

4. Verfahren nach Anspruch 1 oder Anspruch 2, wobei das oder die Gruppe IB Metalle Silber umfassen.

5. Verfahren nach Anspruch 1 oder Anspruch 2, wobei das oder die Gruppe VIII Metalle Palladium umfassen.

6. Verfahren nach Anspruch 1 oder Anspruch 2, wobei das oder die Gruppe VIII Metalle Platin umfassen.

7. Verfahren nach Anspruch 1 oder Anspruch 2, wobei das oder die Gruppe IB Metalle Kupfer oder Silber umfassen und wobei das oder die Gruppe VIII Metalle Platin umfassen.

8. Verfahren nach Anspruch 7, wobei die Gruppe IB und Gruppe VIII Metalle aus Kupfer und Platin bestehen.

9. Verfahren nach einem der vorhergehenden Ansprüche, wobei der Katalysator von 0,01 bis 5,0 Gew.-% Gruppe VIII Metall oder Verbindung auf elementarer Basis und von 0,01 bis 20 Gew.-% Gruppe IB Metall oder Verbindung, ebenfalls auf elementarer Basis, umfaßt.

10. Verfahren nach Anspruch 9, wobei der Katalysator von 0,03 bis 3,0 Gew.-% Gruppe VIII Metall oder Verbindung auf elementarer Basis und von 0,05 bis 15 Gew.-% Gruppe IB Metall oder Verbindung, ebenfalls auf elementarer Basis, umfaßt.

11. Verfahren nach Anspruch 10, wobei der Katalysator von 0,05 bis 1,0 Gew.-% Gruppe VIII Metall oder Verbindung auf elementarer Basis und von 0,1 bis 10 Gew.-% Gruppe IB Metall oder Verbindung, ebenfalls auf elementarer Basis, umfaßt.

12. Verfahren nach einem der vorhergehenden Ansprüche, wobei der Katalysator-Träger ein Kohlenstoff mit einer spezifischen Oberfläche von mindestens 200 m²/g ist.

13. Verfahren nach Anspruch 12, wobei der Katalysator-Träger ein Kohlenstoff mit einer spezifischen Oberfläche von mindestens 400 m²/g ist.

14. Verfahren nach Anspruch 13, wobei der Katalysator-Träger ein Kohlenstoff mit einer spezifischen Oberfläche von mindestens 600 m²/g ist.

15. Verfahren nach einem der vorhergehenden Ansprüche, wobei das Einsatzmaterial ausgewählt wird aus 2-Chlorpropan, 1,2-Dichlorpropan und 1,2,3-Trichlorpropan und Gemischen davon.

## Revendications

1. Procédé de conversion d'une charge d'alimentation à base d'alcane chloré en produits de réaction comprenant un alcène moins chloré, au moyen d'une réaction avec de l'hydrogène en présence d'un catalyseur sur support, caractérisé en ce que le catalyseur est constitué d'un ou plusieurs métaux du groupe IB sous forme élémentaire ou composée et d'un ou plusieurs métaux du groupe VIII choisis parmi le platine, le palladium, l'iridium et le ruthénium sous forme élémentaire ou composée sur le support.

2. Procédé selon la revendication 1, dans lequel du chlorure d'hydrogène est incorporé dans l'alimentation du procédé.

3. Procédé selon l'une des revendications 1 ou 2, dans lequel ledit un ou plusieurs métaux du groupe IB comprend le cuivre.

4. Procédé selon l'une des revendications 1 ou 2, dans lequel ledit un ou plusieurs métaux du groupe IB comprend l'argent.

5. Procédé selon l'une des revendications 1 ou 2, dans lequel ledit un ou plusieurs métaux du groupe VIII comprend le palladium.

6. Procédé selon l'une des revendications 1 ou 2, dans lequel ledit un ou plusieurs métaux du groupe VIII comprend le platine.

7. Procédé selon l'une des revendications 1 ou 2, dans lequel ledit un ou plusieurs métaux du groupe IB comprend le cuivre ou l'argent et dans lequel ledit un ou plusieurs métaux du groupe VIII comprend le platine.

8. Procédé selon la revendication 7, dans lequel les métaux du groupe IB et du groupe VIII sont le cuivre et le platine.

9. Procédé selon l'une des revendications précédentes, dans lequel le catalyseur comprend un métal ou un composé de métal du groupe VIII à raison de 0,01 à 5,0% en poids sur une base élémentaire et un métal ou un composé de métal du groupe IB à raison de 0,01 à 20% en poids, également sur une base élémentaire.

10. Procédé selon la revendication 9, dans lequel le catalyseur comprend un métal ou un composé de métal du groupe VIII à raison de 0,03 à 3,0% en poids sur une base élémentaire et un métal ou un composé de métal du groupe IB à raison de 0,05 à 15% en poids, également sur une base élémentaire.

11. Procédé selon la revendication 10, dans lequel le catalyseur comprend un métal ou un composé de métal du groupe VIII à raison de 0,05 à 1,0% en poids sur une base élémentaire et un métal ou un composé de métal du groupe IB à raison de 0,1 à 10% en poids, également sur une base élémentaire.

12. Procédé selon l'une des revendications précédentes, dans lequel le support du catalyseur est un carbone ayant une surface spécifique d'au moins 200 m²/g.

13. Procédé selon la revendication 12, dans lequel le support du catalyseur est un carbone possédant une surface spécifique d'au moins 400 m²/g.

14. Procédé selon la revendication 13, dans lequel le support du catalyseur est un carbone possédant une surface spécifique d'au moins 600 m²/g.

15. Procédé selon l'une des revendications précédentes, dans lequel la charge d'alimentation est choisie parmi le 2-chloropropane, le 1,2-dichloropropane et le 1,2,3-trichloropropane et les mélanges de ceux-ci.
